# EUROPÄISCHE PATENTSCHRIFT

(11) **EP 0 529 641 B1**
(45) Veröffentlichungstag und Bekanntmachung des Hinweises auf die Patenterteilung: **03.07.1996**
(21) Anmeldenummer: 92114674.2
(22) Anmeldetag: 27.08.1992
(51) Int. Cl.: A61F 13/15

(54) **Wegwerf-Erziehungshöschen**
Disposable training pants
Culotte d'apprentissage jetable

(30) Priorität: 28.08.1991 JP 242776/91
(43) Veröffentlichungstag der Anmeldung: 03.03.1993
(73) Patentinhaber: UNI-CHARM CORPORATION, Kawanoe-shi Ehime-ken (JP)
(72) Erfinder: Kitaoka, Hideaki, Funabashi-shi, Chiba-ken (JP); Sasaki, Tohru, Kawanoe-shi, Ehime-ken (JP)
(74) Vertreter: Sperling, Rüdiger, Dipl.-Ing.

(56) Entgegenhaltungen:
- WO-A-91/11161
- GB-A- 2 023 067
- US-A- 4 960 414

## Beschreibung

### DER ERFINDUNG ZUGRUNDELIEGENDER STAND DER TECHNIK

Die vorliegende Erfindung betrifft Wegwerf-Erziehungshöschen und insbesondere sogenannte Erziehungshöschen, die zur Erziehung von Babys verwendet werden, um sie möglichst frühzeitig an ein Leben ohne Windeln zu gewöhnen (siehe WO-A-91/11161).

Herkömmliche Erziehungshöschen bestehen im allgemeinen aus Stoff und werden nach dem Waschen wieder verwendet. Sondert ein Baby, das dieses Höschen trägt, Ausscheidungen ab, so tritt unmittelbar eine gewisse Menge flüssiger Ausscheidungen aus dem Höschen aus. Demzufolge fühlen sich die Babys unwohl und informieren ihre Mütter über diese Ausscheidungen.

Derartige Erziehungshöschen sind jedoch nicht nur insofern nachteilhaft, als sie gewaschen werden müssen, sondern auch dadurch, daß Teppiche oder ähnliche Bodenbeläge oftmals durch die aus den Höschen austretenden Exkremente verunreinigt werden.

Demgemäß ist es eine Hauptaufgabe der Erfindung, Höschen aufzuzeigen, die dergestalt verbessert sind, daß kein Austreten von flüssigen Ausscheidungen auftritt, was bei den herkömmlichen, aus Stoff bestehenden Höschen unvermeidbar war, und die andererseits in wirksamer Weise den Babys bewußt machen, daß die Höschen durch Exkremente bzw. Ausscheidungen benetzt wurden.

### BESCHREIBUNG DER ERFINDUNG

Erfindungsgemäß wird vorstehende Aufgabe durch Wegwerf-Erziehungshöschen gelöst, die eine flüssigkeitsdurchlassige obere Lage, eine flüssigkeitsundurchlässige untere Lage und einen zwischen die obere und die untere Lage eingelegten flüssigkeitsabsorbierenden Kern umfassen und eine Hüftöffnung und zwei Beinöffnungen aufweisen, wobei sowohl die Hüftöffnung als auch die Beinöffnungen jeweils mit dehnbaren elastischen Elementen versehen sind, wobei die Wegwerf-Erziehungshöschen dadurch gekennzeichnet sind, daß mit der oberen Lage in wenigstens einem zentralen Bereich derselben eine feuchtigkeitsrückhaltende Lage teilweise verbunden ist, so daß die feuchtigkeitsrückhaltende Lage teilweise schwimmend bzw. abgehoben auf der oberen Lage liegt.

Vorzugsweise umfaßt die feuchtigkeitsrückhaltende Lage Vliesstoff, der im wesentlichen aus hydrophilen Fasern hergestellt ist. Alternativ kann die feuchtigkeitsrückhaltende Lage aus weichem Schwammaterial hergestellt sein. Die obere Lage ist dehnungselastisch und die schwimmenden Bereiche sind durch teilweises Verbinden der feuchtigkeitsrückhaltenden Lage mit der oberen Lage gebildet, während die obere Lage gedehnt wird.

Bei den Erziehungshöschen gemäß dieser Erfindung mit dem vorstehend beschriebenen Aufbau werden flüssige Ausscheidungen zunächst von der feuchtigkeitsrückhaltenden Lage absorbiert und eine bestimmte Menge derartiger flüssiger Ausscheidungen, die einmal in den schwimmenden bzw. abgehobenen Bereichen der feuchtigkeitsrückhaltenden Lage absorbiert sind, ist im wesentlichen der direkten Absorptionswirkung des zwischen der oberen Lage und der unteren Lage gelegten Kernes nicht ausgesetzt, so daß diese Menge der flüssigen Ausscheidungen im wesentlichen in den abgehobenen Bereichen verbleibt. Dadurch machen diese abgehobenen Bereiche den Babys die Feuchtigkeit bewußt.

### KURZE BESCHREIBUNG DER ZEICHNUNG

Nachfolgend werden anhand eines Beispiels unter Bezug auf die beiliegenden Figuren die erfindungsgemäßen Erziehungshöschen erläutert. Es zeigen:
- Fig. 1: eine perspektivische Darstellung einer Ausführungsform der gemaß vorliegender Erfindung aufgebauten Erziehungshöschen;
- Fig. 2: eine teilweise ausgebrochene perspektivische Darstellung der Innenseite des entfalteten Höschens; und
- Fig. 3: eine vergrößerte Schnittdarstellung entlang der Linie X-X in Fig. 2.

### BESCHREIBUNG DER BEVORZUGTEN AUSFÜHRUNGSFORMEN

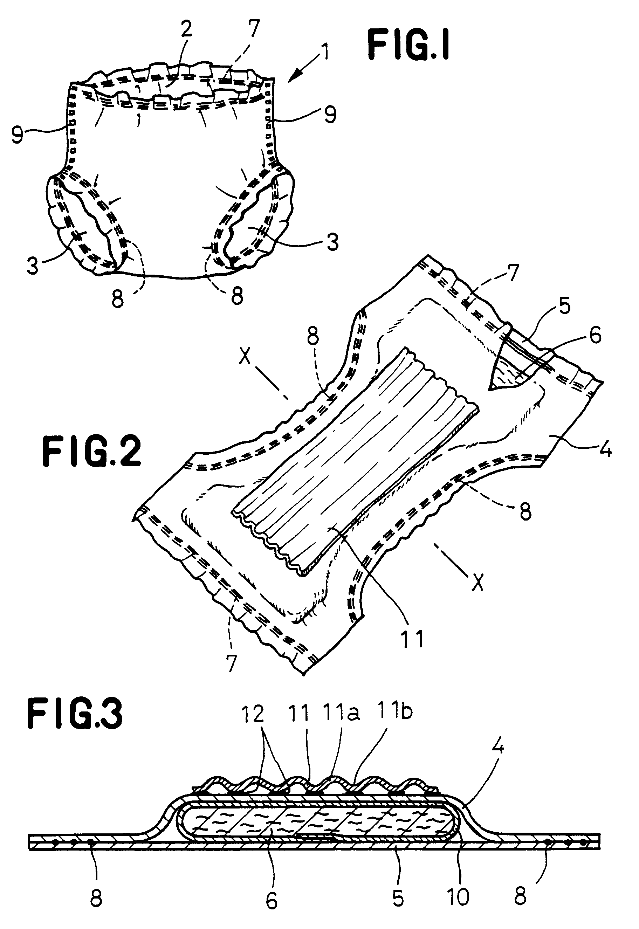

Ein in Fig. 1 bis Fig. 3 gezeigtes Höschen 1 hat eine Hüftöffnung 2 und zwei Beinöffnungen 3. Das Höschen 1 umfaßt eine flüssigkeitsdurchlässige obere Lage 4, eine flüssigkeitsundurchlässige untere Lage 5 und einen lagen- oder mattenähnlichen flüssigkeitsabsorbierenden Kern 6, der zwischen die obere und untere Lage gelegt ist. Dehnbare elastische Elemente 7, 8 sind mittels Klebstoff in gestrecktem Zustand zwischen die Bereiche der oberen und unteren Lage 4, 5 geklebt, die die Hüftöffnung 2 bzw. die Beinöffnungen 3 umgeben. Die Einzelteile des Höschens 1 sind schichtweise übereinandergelegt, wobei die obere Lage 4 im entfalteten Zustand, wie in Fig. 2 dargestellt, an der Innenseite liegt. Dieser Schichtkörper wird in Längsrichtung übereinandergefaltet und punktweise entlang den gegenüberliegenden Seiten verschweißt, wobei die jeweiligen Beinöffnungen 3 unverschweißt bleiben. Wenigstens in einem zentralen Bereich der oberen Lage 4 ist mit dieser eine feuchtigkeitsrückhaltende Lage 11 verbunden.

Die obere Lage besteht aus Vliesstoff, der aus thermoplastischen, hydrophoben, gekräuselten Fasern, wie etwa Polypropylen oder Polyester hergestellt ist, oder aus derartigen Fasern, die nach Wunsch mit geeigneten hydrophilen Fasern, beispielsweise Rayon oder Acetatfasern in einem Gewichtsverhältnis von 0 bis 50 % gemischt werden können und auf jeden Fall einem Verschlingungs- oder Verwicklungsverfahren durch Flüssigkeitsstrahlen unterzogen werden, um so eine Lage zu bilden, die sowohl in der Länge als auch in der Breite dehnbar ist und ein spezifisches Gewicht von 25 bis 45 g/m², eine Rohbauschigkeit von 0,2 bis 0,7 mm und eine Dichte von 0,06 bis 0,12 g/cm³ aufweist. Die untere Lage 5 besteht einerseits aus flüssigkeitsdurchlässigem Vliesstoff, der im wesentlichen der oberen Lage 4 ähnlich ist und ebenfalls in Länge und Breite dehnbar ist, sowie andererseits aus einer feuchtigkeitsdurchlässigen Folie, die aus Kunststoff- oder Elastomermaterial hergestellt ist, das ebenfalls in Länge und Breite dehnbar ist und integral mit dem Vliesstoff verbunden ist. Alternativ kann die untere Lage flüssigkeitsundurchlässigen oder flüssigkeitswiderstandsfähigen Vliesstoff umfassen. Der Kern 6 besteht aus einer Mischung von Faserpulpe, thermoplastischen hydrophoben Kräuselfasern und hochabsorbierendem Polymerpulver, die anschließend zu einer Lage oder Matte geformt wird und mit wasserabsorbierendem Seidenpapier 10 bedeckt wird.

Die flüssigkeitsrückhaltende Lage 11 besteht aus Vliesstoff, der aus hydrophilen Fasern, wie etwa Rayon- oder Acetatfasern (einschließlich Polyesterfasern oder ähnliche mit hydrophilisierter Oberfläche) hergestellt ist oder aus derartigen Fasern, die nach Wunsch mit geeigneten hydrophoben Fasern gemischt sind, beispielsweise Polypropylen- oder Polyesterfasern und in jedem Fall einem Schmelzverbinde- oder Flüssigkeitsstrahlverschlingungs- oder Verwirbelungsverfahren unterzogen wurden, um so eine Lage mit einem spezifischen Gewicht von 20 bis 40 g/m², einer Rohbauschigkeit von 0,2 bis 0,1 mm und einer Dichte von 0,025 bis 0,12 g/cm³ zu bilden. Die feuchtigkeitsrückhaltende Lage 11 hat vorzugsweise eine Breite von 40 bis 100 mm und eine Länge von 60 bis 240 mm.

Grundsätzlich hängt es von einer erwünschten relativen Steuerung der Benetzungseigenschaften und Durchlässigkeit der oberen Lage 4 wie auch der feuchtigkeitsrückhaltenden Lage 11 für die Ausscheidungen ab, ob der als obere Lage 4 verwendete Vliesstoff aus hydrophoben Fasern, gemischt mit hydrophilen Fasern hergestellt werden sollte oder nicht, ob der als feuchtigkeitsrückhaltende Lage 11 verwendete Vliesstoff aus hydrophilen Fasern im Gemisch mit hydrophoben Fasern hergestellt werden sollte oder nicht und auf welche Werte das spezifische Gewicht, Rohbauschigkeit und Dichte der oberen Lage 4 und der feuchtigkeitsrückhaltenden Lage 11 jeweils eingestellt werden sollten. Hinsichtlich der Benetzungseigenschaften sollte beispielsweise die feuchtigkeitsrückhaltende Lage 11 vorzugsweise eine höhere Benetzungseigenschaft als die obere Lage 4 in Anbetracht der besonderen Funktion aufweisen, die von dieser feuchtigkeitsrückhaltenden Lage 11 erwartet wird. Es wurde festgestellt, daß die vorstehend erwähnten Werte im allgemeinen befriedigende Ergebnisse beim Erzielen der Aufgabe der Erfindung bieten, ohne daß eine nennenswerte Kostenzunahme eintritt. Es sei jedoch angemerkt, daß die genannten Werte nur als Beispiele genannt wurden und die Erfindung nicht auf diese Werte beschränkt ist.

Eine nicht dargestellte Ausführungsform der feuchtigkeitsrückhaltenden Lage 11 ist aus einem Verbundvliesstoff gebildet, der aus einer oberen Lage mit einer relativ hohen Faserdichte und einer unteren Lage mit einer relativ niedrigen Faserdichte hergestellt ist, oder aus Vliesstoff, der mit einer mit Faserflausch versehenen Unterfläche ausgerüstet ist, um einen weiter verbesserten Feuchtigkeitsrückhalteeffekte zu erzielen. Alternativ kann die feuchtigkeitsrückhaltende Lage 11 aus einer flüssigkeitsabsorbierenden, weichen, schwammigen Lage aus Urethan, Zellulose oder ähnlichem mit einer Stärke von 0,3 bis 2 mm gebildet sein.

Bevor die feuchtigkeitsrückhaltende Lage 11 auf die obere Lage 4 aufgebracht wird, wird die feuchtigkeitsrückhaltende Lage 11 in Wellen gelegt, um so abgehobene Bereiche (Erhebungen) 11a und anliegende Bereiche (Vertiefungen) 11b zu bilden. Die anliegenden Bereich 11b werden mittels eines Verbindungsmittels 12, das an den entsprechenden Bereichen der oberen Lage 4 vorgesehen ist, mit der oberen Lage verbunden. Eine derartige Verbindung kann beispielsweise dadurch erzielt werden, daß die feuchtigkeitsrückhaltende Lage 11 mit der oberen Lage 4 verbunden wird, während diese in Länge und/oder Breite gestreckt wird. Die abgehobenen Bereiche 11a können auch in einem seitlichen Streifenmuster, in Form von verteilten Punkten oder in ähnlicher Weise anstelle der dargestellten vertikalen Streifen angeordnet sein. In jedem Fall sind die abgehobenen Bereiche 11a vorzugsweise in einer möglichst großen Höhe zur oberen Fläche der oberen Lage 4 beabstandet und haben vorzugsweise einen möglichst hohen Flächenanteil bezüglich der gesamten feuchtigkeitsrückhaltenden Lage 11. Genauer beträgt diese Höhe nach dem Zusammenziehen der oberen Lage 4 vorzugsweise 1 mm oder mehr und der Anteil vorzugsweise 20 % oder mehr. Die obere Lage kann teilweise mit dem Verbindungsmittel 12 an den entsprechenden Bereichen versehen sein, abhängig von dem jeweiligen Muster der abgehobenen Bereiche 11a. Die Verbindung kann durch die Verwendung von Klebstoff oder durch Schweißung erfolgen.

Bei dem erfindungsgemäß vorgeschlagenen Erziehungshöschen ist eine einmal in die abgehobenen Bereiche der feuchtigkeitsrückhaltenden Lage aufgenommene Menge von flüssigen Ausscheidungen im wesentlichen nicht von der direkten absorbierenden Wirkung des zwischen der oberen und unteren Lage liegenden Kerns betroffen, so daß diese Menge der flüssigen Ausscheidungen im wesentlichen in den abgehobenen Bereichen verbleibt, die normalerweise mit der Haut der Babys in Berührung stehen, und so das Wohlgefühl der Babys beeinträchtigt. Somit werden die Babys ihrer eigenen Ausscheidungen bewußt und nehmen die Gewohnheit an, eine andere Person über Ausscheidungen während der Verwendung des Höschens zu informieren.

Des weiteren ragen die abgehobenen Bereiche der feuchtigkeitsrückhaltenden Lage normalerweise von der oberen Lage nach oben gegen die Haut der Babys vor. Dieses Merkmal erlaubt es, daß Babys die Feuchtigkeit mit einer höheren Sensibilität empfinden, als das bei nicht vorhandenen abgehobenen Bereichen der Fall ist, auch wenn das Höschen nicht hoch genug am Körper des Babys getragen werden, um die obere Lage im Schrittbereich mit der Haut des Babys in Berührung zu halten, oder auch wenn die Höschen in gewissem Ausmaß aus dieser Ebene verlagert wurden.

Da die feuchtigkeitsrückhaltende Lage in einem Bereich angeordnet ist, der mit größter Wahrscheinlichkeit Ausscheidungen ausgesetzt ist, wird als zweiter Effekt das Anhaften von Exkrementen an der Haut des Babys in wirksamer Weise verringert, da derartige weiche Exkremente von den abgehobenen Bereichen nach unten in die anhaftenden Bereiche fließen.

## Patentansprüche

1. Wegwerf-Erziehungshöschen, umfassend eine flüssigkeitsdurchlässige obere Lage, eine flüssigkeitsundurchlässige untere Lage und einen flüssigkeitsabsorbierenden Kern, der zwischen die obere und untere Lage gelegt ist, sowie eine Hüftöffnung und zwei Beinöffnungen, die jeweils mit dehnbaren elastischen Elementen versehen sind, dadurch gekennzeichnet, daß eine feuchtigkeitsrückhaltende Lage mit der oberen Lage wenigstens in einem zentralen Bereich derselben teilweise verbunden ist, so daß die feuchtigkeitsrückhaltende Lage teilweise von der oberen Lage abgehoben ist und wobei die obere Lage dehnungselastisch ist und die abgehobenen Bereiche durch teilweises Verbinden der feuchtigkeitsrückhaltenden Lage mit der oberen Lage gebildet werden, während die obere Lage gedehnt ist.

2. Erziehungshöschen nach Anspruch 1, wobei die feuchtigkeitsrückhaltende Lage Vliesstoff umfaßt, der vorzugsweise aus hydrophilen Fasern hergestellt ist.

3. Erziehungshöschen nach Anspruch 1, wobei die feuchtigkeitsrückhaltende Lage aus weichem schwammartigen Material hergestellt ist.

4. Verfahren zum Herstellen von Wegwerf-Erziehungshöschen, umfassend eine flüssigkeitsdurchlässige obere Lage, eine flüssigkeitsundurchlässige untere Lage und einen flüssigkeitsabsorbierenden Kern, der zwischen die obere und untere Lage gelegt ist, sowie eine Hüftöffnung und zwei Beinöffnungen, die jeweils mit dehnbaren elastischen Elementen versehen sind, dadurch gekennzeichnet, daß eine feuchtigkeitsrückhaltende Lage mit der oberen Lage wenigstens in einem zentralen Bereich derselben teilweise verbunden ist, so daß die feuchtigkeitsrückhaltende Lage teilweise von der oberen Lage abgehoben ist und wobei die obere Lage dehnungselastisch ist und die abgehobenen Bereiche durch teilweises Verbinden der feuchtigkeitsrückhaltenden Lage mit der oberen Lage gebildet werden, während die obere Lage gedehnt ist.

## Claims

1. Disposable training panties, comprising an upper layer permeable to liquid, a lower layer impermeable to liquid and a liquid-absorbing core which is placed between the upper and lower layers, as well as a hip opening and two leg openings, which are provided with respective stretchable elastic elements, characterized in that a moisture-retaining layer is bonded in places to the upper layer at least in a central region thereof, so that the moisture-retaining layer is raised in places from the upper layer, and wherein the upper layer is elastically stretchable and the raised regions are formed by bonding the moisture-retaining layer in places to the upper layer while the upper layer is stretched.

2. Training panties according to claim 1, wherein the moisture-retaining layer comprises fleece material which is preferably made from hydrophilic fibres.

3. Training panties according to claim 1, wherein the moisture-retaining layer is made from soft, sponge-like material.

4. A method of making disposable training panties, comprising an upper layer permeable to liquid, a lower layer impermeable to liquid and a liquid-absorbing core which is placed between the upper and lower layers, as well as a hip opening and two leg openings, which are provided with respective stretchable elastic elements, characterized in that a moisture-retaining layer is bonded in places to the upper layer at least in a central region thereof, so that the moisture-retaining layer is raised in places from the upper layer, and wherein the upper layer is elastically stretchable and the raised regions are formed by bonding the moisture-retaining layer in places to the upper layer while the upper layer is stretched.

## Revendications

1. Petite culotte jetable d'apprentissage à la propreté, comportant une couche supérieure perméable aux liquides, une couche inférieure imperméable aux liquides et un corps central absorbant les liquides, inséré entre les couches supérieure et inférieure, ainsi qu'une ouverture de hanches et deux ouvertures de passage des jambes, respectivement munies d'éléments élastiques extensibles, caractérisée en ce qu'une couche de rétention d'humidité est raccordée partiellement à la couche supérieure, au moins dans une zone centrale de cette dernière, de telle façon que la couche de rétention d'humidité soit partiellement décollée de la couche supérieure, petite culotte dans laquelle la couche supérieure est élastique sous extension, et les zones décollées sont formées en joignant partiellement la couche de rétention d'humidité à la couche supérieure, pendant que la couche supérieure est étirée.

2. Petite culotte selon la revendication 1, dans laquelle la couche de rétention d'humidité comporte un non tissé fabriqué, de préférence, à base de fibres hydrophiles.

3. Petite culotte selon la revendication 1, dans laquelle la couche de rétention d'humidité est fabriquée dans un matériau moelleux et spongieux.

4. Procédé de fabrication de petites culottes jetables d'apprentissage à la propreté, comportant une couche supérieure perméable aux liquides, une couche inférieure imperméable aux liquides et un corps central absorbant les liquides, inséré entre les couches supérieure et inférieure, ainsi qu'une ouverture de hanches et deux ouvertures de passage des jambes, respectivement munies d'éléments élastiques extensibles, caractérisé en ce qu'une couche de rétention d'humidité est raccordée partiellement à la couche supérieure, au moins dans une zone centrale de cette dernière, de telle façon que la couche de rétention d'humidité soit partiellement décollée de la couche supérieure, petite culotte dans laquelle la couche supérieure est élastique sous extension, et les zones décollées sont formées en joignant partiellement la couche de rétention d'humidité à la couche supérieure, pendant que la couche supérieure est étirée.
